# EUROPEAN PATENT APPLICATION

(11) **EP 4 613 317 A1**
(43) Date of publication of application: **10.09.2025**
(21) Application number: 24161329.8
(22) Date of filing: 05.03.2024
(51) Int. Cl.: A61M 25/01, A61B 18/14

(54) **HANDLE FOR BIDIRECTIONAL DEFLECTION OF A CATHETER**

(71) Applicant: VascoMed GmbH, 79589 Binzen (DE)
(72) Inventor: Kaufmann, Ralf, 79540 Lörrach (DE)
(74) Representative: Biotronik Corporate Services SE

(57) **Abstract**

The present invention relates to a catheter (1), comprising: a shaft (12) having a deflectable section (11), and a handle (45) connected to the shaft (12), wherein the handle (45) comprises a rim (20) having a toothed portion (20a) and a rotatable actuating member (200) configured to be rotated to deflect the deflectable section (11), wherein the handle (45) further comprises a first control member (8a) coupled to a spring member (17) having a first toothed portion (19a) configured to engage with the toothed portion (20a) of the rim (20) to prevent rotation of the actuating member (200), and wherein the first control member (8a) is configured to be manually moved from a first position to a second position to deform the spring member (17) such that the first toothed portion (19a) of the spring member (17) disengages with the toothed portion (20a) of the rim (20) to allow rotation of the actuating member (200) for deflecting the deflectable section (11) of the shaft (12).

## Description

The present invention relates to a catheter. Particularly, the present invention relates to a mechanism as a component of a handle for bidirectionally steerable catheters.

Known solutions of such handles that can be operated to deflect a distal section of a catheter often require either the second hand or sometimes an uncomfortable thumb or index finger movement to actuate a friction-based parking brake to set the deflection position.

Based on the above, the problem to be solved by the invention at hand is to provide a catheter with a handle comprising an improved operability regarding adjustment of a deflection of section of a shaft of the catheter. Particularly, it is desirable to provide a handle that only requires a slight lateral touch of one or two control members with the intention of a rotation to achieve a deflection in the direction of rotation that is sufficient to unlock a control gear. Ideally, only the thumb and/or index finger of one hand is required to control (deflect) the catheter.

This problem is solved by a catheter having the features of claim 1. A further aspect of the present invention relates to a catheter comprising at least one elongated pulling member for deflecting the deflectable section of the catheter. Preferred embodiments of these aspects of the present invention are stated in the corresponding dependent claims and are described below.

According to claim 1, a catheter is disclosed, comprising:
- a shaft having a deflectable section, and
- a handle connected to the shaft, wherein the handle comprises a rim having a toothed portion and a rotatable actuating member configured to be rotated to deflect the deflectable section, wherein the handle further comprises a first control member coupled to a spring member having a first toothed portion configured to engage with the toothed portion of the rim to prevent rotation of the actuating member, and wherein the first control member is configured to be manually moved from a first position to a second position to deform the spring member such that the first toothed portion of the spring member disengages with the toothed portion of the rim to allow rotation of the actuating member for deflecting the deflectable section of the shaft.

Particularly, via the at least one (first) control member, the spring member is sensitive to touch pressure. Contact pressure on the control member(s) (e.g. control lever(s)) may transform an elliptical spring member into a circular shape. As a result, a small semi-axis of the spring member enlarges and, with it, can lift two opposing internal toothed portions off the toothed portion of the stationary rim.

In the framework of the present invention, particularly, toothed portions of the spring member and rim that engage with one another are understood to be any structures that allow engagement in a radial direction of the spring member to then prevent a rotation of the spring member about its rotation axis due to this engagement, wherein the rotation axis runs perpendicular to the radial direction.

Thus, in an embodiment, a toothed portion of the rim can comprise a plurality of adjacent protrusions arranged side by side in a peripheral direction of the rim that can interlock with at least one or a plurality of protrusions of an opposing toothed portion of the spring member. In embodiments, the toothed portion of the rim can extend along the whole periphery of the rim, particularly on an outside of the rim that faces an inside of the spring member. However, there are also embodiments, where the toothed portion of the rim can extend merely along a section of the periphery of the rim.

Particularly, when the toothed portions of rim and spring member disengage by actuation of the control member(s), rotation of the spring member is permitted without resistance, and can actuate a deflection mechanism of the handle allowing to deflect the deflectable section of the shaft in one of two opposite deflection directions depending on the rotation direction of the spring member / actuating member. Furthermore, particularly, in an embodiment, releasing the control member(s) allows the spring member to spring back into its initial (e.g. elliptical) shape so that the toothed portions of spring member and rim re-engage to prevent further rotation of the actuating member. With the spring member thus locked again against rotation, the deflection mechanism of the handle is also locked. The deflection achieved is held in its set position.

Thus, the present invention allows to achieve an auto-lock function without friction. The set deflection of the catheter is locked stably when the control member(s) is (are) released. A slight actuation of the control member(s) unlocks the auto-lock function immediately and a different deflection shape can be set. Particularly, the mechanism can be unlocked by light pressure when the control member(s) is (are) touched.

According to an embodiment of the present invention, the spring member comprises an annular part comprising an inside delimiting an opening of the spring member, wherein the opening accommodates the rim, and wherein the first toothed portion of the spring member is arranged on the inside and protrudes radially inwards and can face the toothed portion of the rim. A second toothed portion can be arranged opposite the first toothed portion and can also protrude radially inwards and can face the toothed portion of the rim.

Particularly, in an embodiment, the opening comprises a first inner diameter and a second inner diameter perpendicular to the first inner diameter, wherein the first toothed portion of the spring member faces the toothed portion of the rim in the direction of the first inner diameter, wherein when the first control member resides in the first position, the first inner diameter is smaller than the second inner diameter, and wherein when the first control member resides in the second position, the annular spring member is deformed so that the first inner diameter is increased to disengage the first toothed portion of the spring member and the toothed portion of the rim and the second inner diameter is decreased.

Furthermore, according to an embodiment of the invention, the first control member is coupled (particularly connected, particularly integrally connected) to the spring member and is configured to be pushed radially inwards from the first position to the second position to deform the annular member so that the first inner diameter increases and the second inner diameter decreases. The spring member can comprise a second control member and a second toothed portion that can operate in the same manner. Particularly, the first and/or second control member can be pushed and moved to their second positions to disengage the first toothed portion and particularly also the second toothed portion from the toothed portion of the rim.

Further, according to yet another embodiment of the present invention, when the first control member (and particularly the second control member) resides in the first position, the inside of the annular part of the spring member comprises a non-circular oval shape, particularly an elliptical shape, and wherein when the first control member (and particularly the second control member) resides in the second position, the spring member is deformed so that said inside comprise a more circular shape to lift the first toothed portion (and particularly the second toothed portion) of the spring member off the toothed portion of the rim.

Furthermore, in an embodiment, the handle comprises a second control member coupled (particularly connected, particularly integrally connected) to the spring member, wherein the spring member comprises a second toothed portion configured to engage with the toothed portion of the rim to prevent rotation of the actuating member, and wherein the second control member is configured to be manually moved from a first position to a second position at the same time as the first control member is moved from its first position to its second position to deform the spring member such that the first and the second toothed portion of the spring member both disengage with the toothed portion of the rim for allowing rotation of the actuating member for deflecting the deflectable section of the shaft. Particularly, the two control members can be configured to be pressed manually by the thumb and index finger or by single thumb or single index finger of a single hand of a person operating the catheter. However, due to the structure of the spring member it suffices to actively push only one of the control members.

According to yet another embodiment of the present invention, the handle comprises a second control member coupled to the spring member, wherein the spring member comprises a second toothed portion, and wherein the toothed portion of the rim is comprised of a first (e.g. quarter) segment associated to the first toothed portion of the spring member and an adjacent second (e.g. quarter) segment associated to the second toothed portion of the spring member. Particularly, the term quarter segment means that the respective segment extends along a periphery of the rim over a length that corresponds to about 90°, thus, particularly, the toothed portion of the rim extends over about half of the circumference of the rim which is otherwise smooth so that the toothed portions of the spring member cannot engage with this smooth portion of the rim.

Particularly, the first toothed portion is configured to engage with the first segment to prevent rotation of the actuating member in a first rotation direction, wherein the second toothed portion is configured to engage with the second segment to prevent rotation of the actuating member in an opposite second rotation direction, and wherein the second control member is configured to be manually moved from a first position to a second position at the same time as the first control member is moved from its first position to its second position to deform the spring member such that the first toothed portion disengages with the first segment or such that the second toothed portion disengages with the second segment of the toothed portion of the rim for allowing rotation of the actuating member for deflecting the deflectable section of the shaft.

Particularly, in an embodiment, when the actuating member is rotated in the first rotation direction (e.g. to generate a left deflection of the deflectable section) and the control members are released to return from the second positions into the first positions, the first toothed portion of the spring member engages with the first segment of the toothed portion of the rim whereas the second toothed portion of the spring member does not engage with the toothed portion of the rim, and wherein when the actuating member is rotated in an opposite second rotation direction (e.g. to generate a right deflection of the deflectable section) and the control members are released to return from the second positions into the first positions, the second toothed portion of the spring member engages with the second segment of the toothed portion of the rim whereas the first toothed portion of the spring member does not engage with the toothed portion of the rim.

According to yet another embodiment of the present invention, the second control member is arranged opposite the first control member. Furthermore, in an embodiment, the second toothed portion of the spring member is arranged opposite the first toothed portion of the spring member.

Furthermore, according to an embodiment of the present invention, the spring member further comprises a force deflection structure connected to the inside of the annular part of the spring member, wherein the force deflection structure is configured to transmit a force introduced into the spring member via one of the control members (when moving the first or second control member from its first position to its second position) to the first and/or second toothed portion of the spring member, thus amplifying the effect of the respective control member.

Furthermore, according to yet another embodiment of the present invention, the force deflection structure contacts a protrusion received in a through-opening of the force deflection structure, wherein the protrusion is connected to the actuating member in a rotationally fixed manner.

Further, in an embodiment, the spring member comprises two opposing guide pins or guide cuboids for guiding the deformation of the spring member when the first and/or second control member is/are moved from the respective first position to the respective second position, wherein particularly the guide pins or guide cuboids protrude from the annular part of the spring member, particularly parallel to the rotation axis of the spring member.

According to an embodiment, the force deflection device comprises four arc-shaped struts that delimit the through-opening of the force deflection structure, wherein each strut extends from a point of the inside in the vicinity of one of the control members towards a point of the inside in the vicinity of one of the guide pins, and - in between these two points - contacts the protrusion received in the through-opening of the force deflection structure. Particularly, at the respective control member and at the respective guide pin two neighboring struts are joined.

According to a further embodiment, the handle comprises an actuating knob coupled to the actuating member for manually rotating the actuating member, wherein particularly the knob comprises a first slot slidably receiving the first control member and an opposing second slot slidably receiving the second control member, and wherein particularly the knob comprises two opposing grooves, each groove receiving one of the guide pins so that the respective guide pin can slide in its associated groove when the first and/or second control member is moved from its first position to its second position and vice versa.

According to yet another embodiment of the present invention, the catheter further comprises an elongated first pulling member connected to the deflectable section for deflecting the deflectable section when pulling on the first pulling member, wherein the actuating member is rotatably supported on a base of the handle (e.g. via a cover of the handle / the housing of the handle) so that the actuating member is rotatable about a rotation axis with respect to the base, and wherein the handle further comprises a first deflection element arranged on the base and a second deflection element arranged on the rotatable actuating member spaced apart from the rotation axis of the latter, wherein the first pulling member coming from the deflectable section is deflected by the first deflection element towards the second deflection element that deflects the first pulling element towards an anchoring of the elongated first pulling member on the base so that the first pulling member forms a pulley and a rotation of the actuating member in a first direction about the rotation axis increases a distance between the first and the second deflection element and generates a pulling force on the first pulling member thereby causing an (e.g. left) deflection of the deflectable section in a first deflection direction.

Furthermore, according to yet another embodiment of the catheter of the present invention, a third deflection element is arranged on the rotatable actuating member opposite the second deflection element and spaced apart from the rotation axis of the actuating member, and wherein the catheter comprises an elongated second pulling member connected to the deflectable section of the shaft of the catheter, which second pulling member - coming from the deflectable section - is deflected by the first deflection element towards the third deflection element that deflects the second pulling element towards a second anchoring of the elongated second pulling member on the base so that the second pulling member forms a pulley and a rotation of the actuating member in a second direction about the rotation axis increases a distance between the first and the third deflection element and generates a pulling force on the second pulling member thereby causing an (e.g. right) deflection of the deflectable section in a second deflection direction.

Furthermore, according to a second aspect of the present invention a catheter is disclosed, comprising:
- a shaft having a deflectable section,
- an elongated first pulling member connected to the deflectable section for deflecting the deflectable section when pulling on the first pulling member, and
- a handle connected to the shaft, wherein the handle comprises a rotatable actuating member being rotatably supported on a base of the handle so that the actuating member is rotatable about a rotation axis with respect to the base, and wherein the handle further comprises a first deflection element arranged on the base and a second deflection element arranged on the rotatable actuating member spaced apart from the rotation axis, wherein the first pulling member coming from the deflectable section is deflected by the first deflection element towards the second deflection element that deflects the first pulling element towards an anchoring of the first pulling member on the base so that the first pulling member forms a pulley in particular and a rotation of the actuating member in a first direction about the rotation axis increases a distance between the first and the second deflection element and generates a pulling force on the first pulling member thereby causing a deflection of the deflectable section in a first deflection direction.

According to an embodiment of the catheter according to the second aspect, the handle comprises a rim having a toothed portion and a first control member coupled (particularly connected, particularly integrally connected) to a spring member having a toothed portion configured to engage with the toothed portion of the rim to prevent rotation of the actuating member, and wherein the first control member is configured to be manually moved from a first position to a second position to deform the spring member such that the toothed portion of the spring member disengages with the toothed portion of the rim to allow rotation of the actuating member for deflecting the deflectable section of the shaft.

Furthermore, the second aspect of the invention may be further characterized by the embodiments described in connection with the first aspect of the invention above, which embodiments also form embodiments of the second aspect of the present invention. The same applies vice versa for the embodiments described in conj unction with the second aspect of the present invention, which in turn can be used to further specify the first aspect of the present invention.

In the following, embodiments of the first and second aspect of the present invention as well as further features and advantages of these aspects and embodiments shall be described in conjunction with the Figures, wherein
- Fig. 1: shows an embodiment of a catheter according to the first and second aspect of the present invention, wherein the catheter comprises a shaft having a deflectable section that is in an undeflected state,
- Fig. 2: shows the embodiment of Fig. 1, wherein the deflectable section is in a right deflected state, whereas the dashed portions indicate a complete left deflection of said deflectable section,
- Fig. 3: shows a perspective view onto the handle of the catheter shown in Figs. 1 and 2,
- Fig. 4: shows an exploded view of the handle shown in Fig. 3,
- Fig. 5a: shows a detail of a handle according to an embodiment of the first and the second aspect of the invention, wherein toothed portions of a spring member of the handle are engaged with a toothed portion of a rim of the handle to lock an actuating member of the handle and thereby lock a deflection mechanism of the handle,
- Fig. 5b: shows a state where the toothed portions of the spring member as shown in Fig. 5a are disengaged with the toothed portion of a rim of the handle to allow rotation of the actuating member of the handle and thereby a corresponding deflection of the deflectable section the catheter shaft,
- Fig. 6: shows a cross-sectional view of the handle along the line A-A of Fig. 3,
- Fig. 7: shows a further cross-sectional view along the handle indicating the course of elongated pulling members that couple the actuating member to the deflectable section,
- Fig. 8: shows the handle of Fig. 7, wherein the actuating member is fully rotated in a first rotation direction corresponding to the full left deflection of the deflectable section shown in Fig. 2 with dashed lines,
- Fig. 9a: shows an embodiment of the spring member of the handle comprising a force deflection means comprising arc-shaped struts contacting a protrusion having a polygonal (e.g., square) cross-section,
- Fig. 9b: shows a modification of the embodiment shown in Fig. 9a, wherein the arc-shaped struts contact a circular cylindrical protrusion, and
- Fig. 10: shows a detail of a further embodiment of the first and second aspect of the present invention, wherein here the toothed portions of the spring member only engage with an associated segment of a toothed portion of the rim depending on rotation direction,

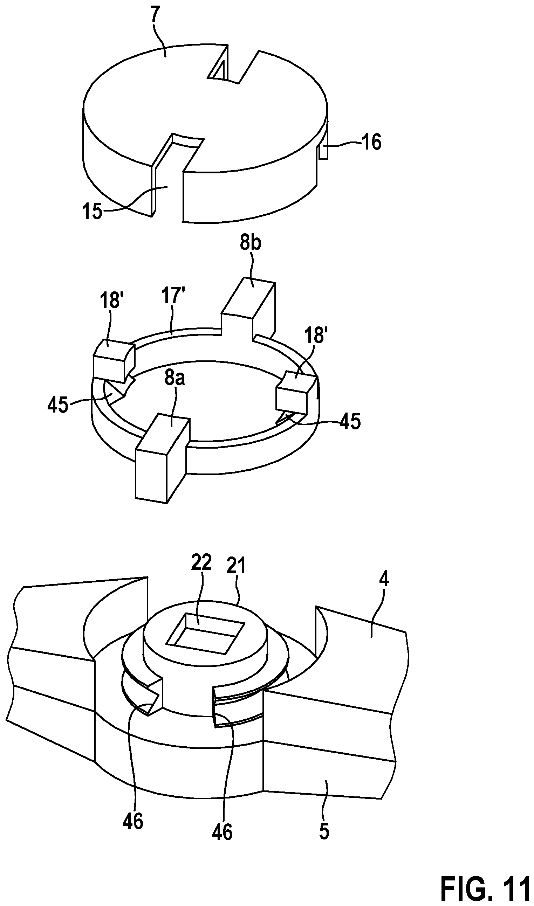

- Fig. 11: shows another exploded view of the handle shown in Fig. 3 and 4, with wedge clamps instead of toothed portions,
- Fig. 12: shows a further cross-sectional view along the handle indicating another arrangement of the course of elongated pulling members that couple the actuating member to the deflectable section suitable for guiding shafts,

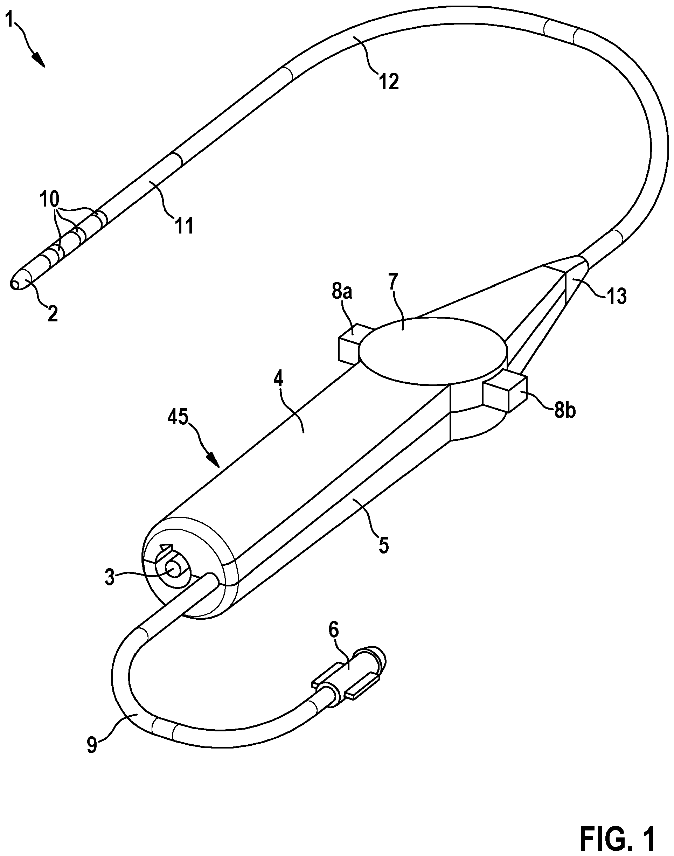

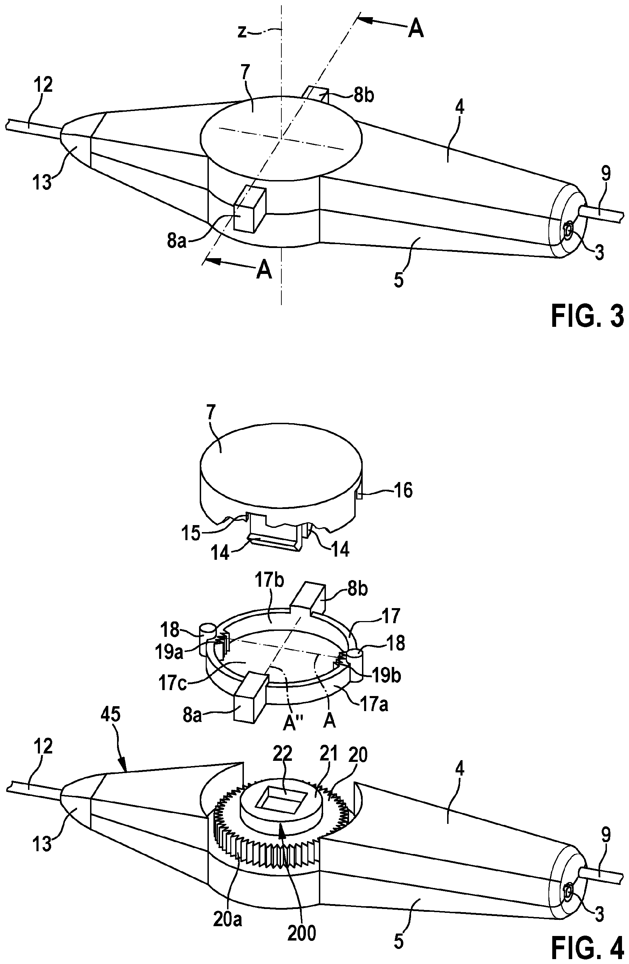

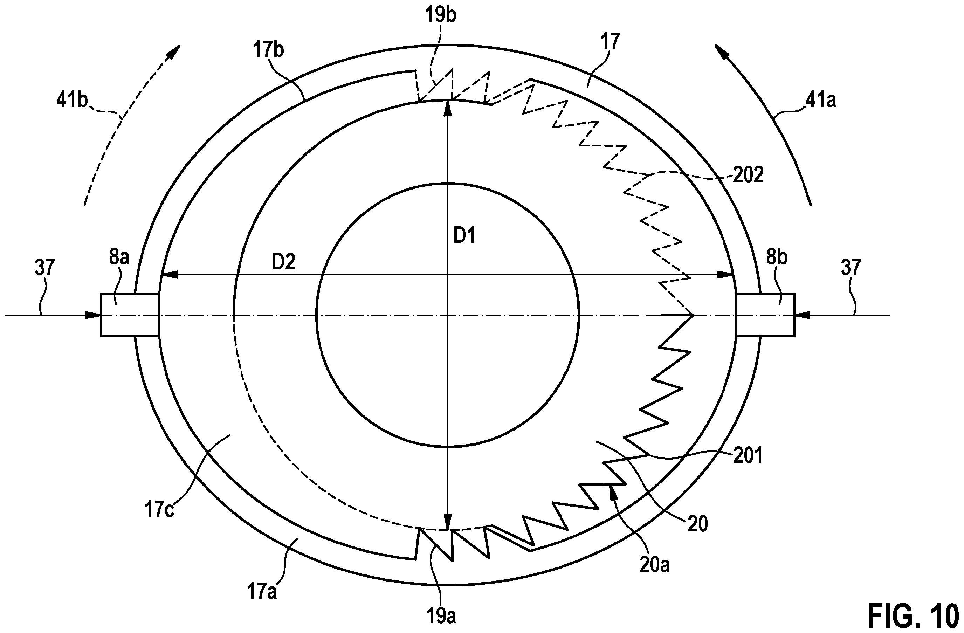

Fig. 1 shows an embodiment of a catheter according to the present invention. The catheter 1 allows precise steering/deflection of a deflectable section 11 of a shaft 12 of the catheter 1 at a distal end of the catheter shaft 12. The catheter 1 further comprises a handle 45 connected to the shaft 12, wherein the handle 45 comprises a rim 20 (cf. e.g. Fig. 4) having an outer toothed portion 20a and a rotatable actuating member 200 configured to be rotated to deflect the deflectable section 11, wherein the handle 45 further comprises a first control member 8a and particularly also a second control member 8b coupled to a spring member 17, respectively. The coupling can be achieved by connecting the control member(s) 8a, 8b to the spring member 17, e.g. integrally. The spring member 17 comprises a first toothed portion 19a and particularly also a second toothed portion 19b configured to engage with the toothed portion 20a of the rim 20, respectively, to prevent rotation of the actuating member 200. Furthermore, the respective control member 8a, 8b is configured to be manually moved from a first position to a second position to deform the spring member 17 such that the respective toothed portion 19a, 19b of the spring member 17 disengages with the toothed portion 20a of the rim 20 to allow rotation of the actuating member 200 about a rotation axis z for deflecting the deflectable section 11 of the shaft 12. Particularly, the toothed portion 20a of the rim 20 can be a circumferential toothed portion as shown in Fig. 4. However, it is also conceivable that only a section of the circumference of the rim 20 is provided with a toothed portion 20a as shown in Fig. 10 which will be described in detail further down below.

Particularly, as indicated in Fig. 1, the catheter 1 can be an electrophysiological bidirectionally steerable catheter 1. Further, the handle 45 can be designed as a so-called "wing handle", which is particularly used for RF or PFA ablations. The bidirectionally deflectable section 11 is located on the shaft 12, at the end of which an ablation electrode 2 and three mapping ring electrodes 10 can be arranged. The shaft 12 can be connected to the handle 45 through a kink protection 13. The handle 45 can comprise a housing (cf. e.g., Figs. 1 to 4) that comprises a handle cover 4 and a handle base 5 that is also briefly denoted as base 5. At a proximal end of the handle housing 4, 5, a plug socket 3 can be provided for a patient cable (not shown) and an optional flushing line 9 with a Luer lock connection 6. In the more distal part of the handle housing 4, 5, the first (e.g. left) control member 8a and the second (e.g. right) control member 8b can be attached so that they protrude (e.g. in the form of levers 8a, 8b) from an e.g. circular rotating knob 7 via which the actuating member 200 can be operated.

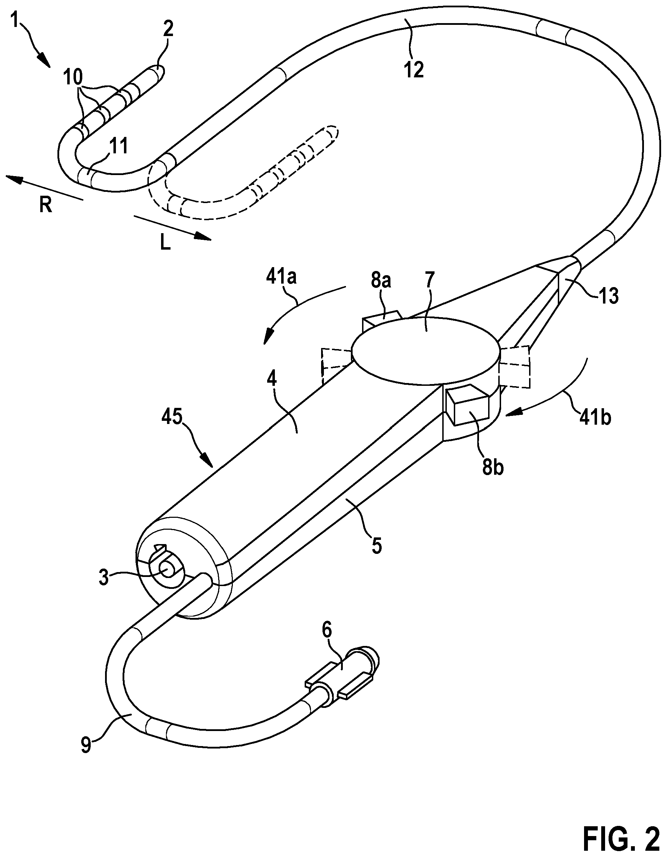

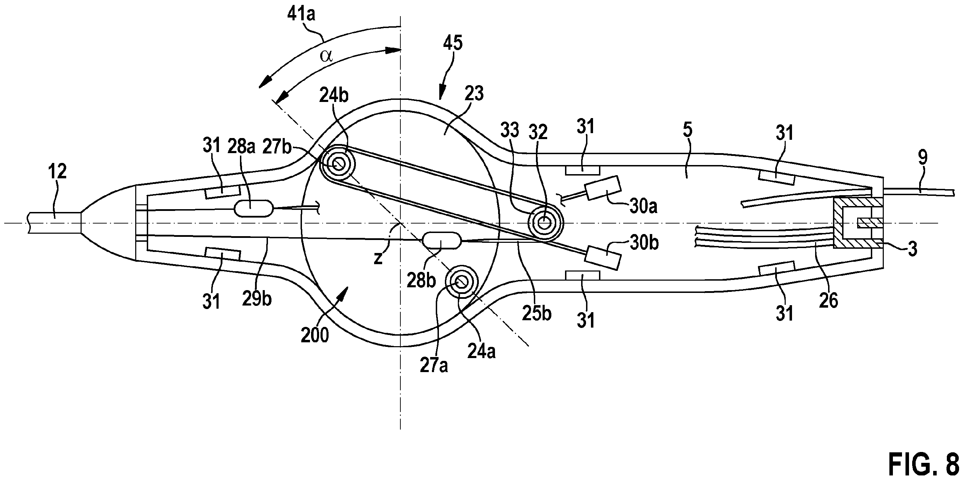

Fig. 2 shows the catheter 1 of Fig. 1 in both maximum deflection states of the section 11, namely deflected to the left in a first deflection direction L (shown in dashed lines) and to the right in a second deflection direction R. Corresponding to this, when the control members 8a, 8b and therewith the actuating member 200 are rotated counter clockwise by an angle α of about 40° in a first rotation direction 41a (cf. also Fig. 8) the left deflection of section 11 is realized, whereas in case the control members 8a, 8b (and thus the actuating member 200) are rotated clockwise from neutral position (non-deflection) by the same amount the maximum right deflection of the deflectable section 11 is realized.

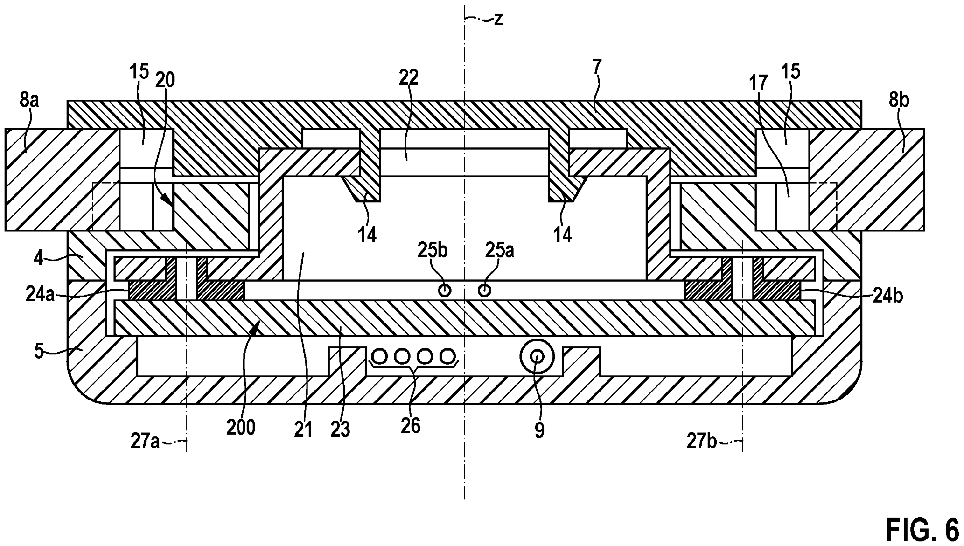

Fig. 3 shows a perspective view of the handle of the catheter 1 of Figs. 1 and 2, with the shaft 12 and the irrigation tube 9, provided with a broken edge, not shown in their entirety. The cross section along A-A is shown in Fig. 6.

Fig. 4 shows the handle 45 of the catheter 1 of Figs. 1 to 3 with the knob 7 lifted off and the spring member 17 lifted off, which corresponds to the condition of the handle 45 as it is before the last assembly step. Furthermore, the rim 20 can form a part of the handle cover 4, particularly an integral part thereof. As shown in Fig. 6, in an embodiment, the actuating member 200 can comprise an upper plate 21 and a lower plate 23. Pushed through from an inside of the handle 45, the upper plate 21 with its rectangular opening 22 is visible.

The spring member 17 can comprise two opposing sets of toothed portions 19a, 19b, e.g., in the form of internal teeth, which toothed portions 19a, 19b can face each other. Further, the spring member 17 can comprise two opposing guide pins 18 (e.g., in form of cylinders or cuboidal structures) facing each other as well and protruding from the spring member 17 in the direction of the rotation axis z. Also, the control members 8a, 8b protrude outwards from the spring member 7. Furthermore, for fixing the knob 7 to the actuating member 200, namely to the upper plate 21, the knob 7 can comprise snap hooks 14. Particularly, in Fig. 4, knob 7 is shown broken out laterally to reveal the snap hooks 14 arranged in pairs. After the spring member 17 has been pushed onto the toothed rim 20, the snap hooks 14 are configured to fix the knob 7 by engaging into opening 22 of the upper plate 21. The two guide pins 18 of the spring member 17 can then move in grooves 16 of the knob 7. Further, the two control members 8a and 8b of the spring member 17 can be configured to move in slots 15 also provided by the knob 7 when actuated. The rotary movement of the actuated control members 8a and/or 8b can be transmitted to the knob 7 via the slots 15 on both sides. The knob 7 in turn transmits its rotation to the actuating member 200 e.g. via the upper plate 21 inside the handle housing 4, 5 (e.g. via the engaged snap hooks 14). Other fastening/connection means to connect the knob 7 to the actuating member 200 are also conceivable.

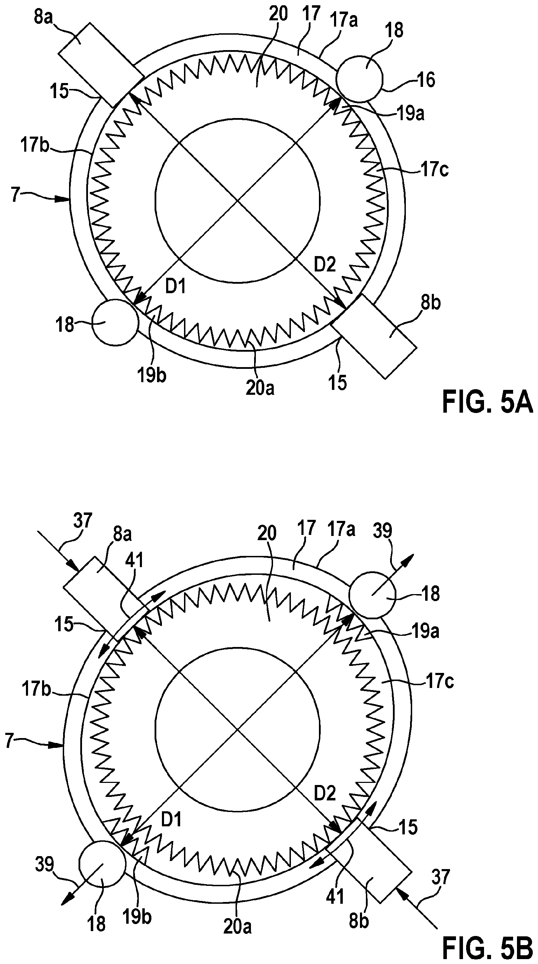

Figures 5a and 5b show an embodiment of a locking mechanism in a locked and unlocked state realized with help of the spring member 7 and rim 20. In the locked state (cf. Fig. 5a) corresponding to a first position of the control members 8a, 8b, the toothed portions 19a, 19b of the annular spring member 17 engage with the toothed portion 20a of the rim 20. To unlock and thus enable the rotation of the knob 7 and actuating member 200 connected thereto about rotation axis z, a slight pressure 37 on the control member(s) 8a or/and 8b is required to move them into a second position and to thereby lift the two toothed portions 19a, 19b off the toothed portion 20a of the rim 20 due to an elastic deformation of the spring member 17. Particularly, the spring member 17 can comprises an annular part 17a comprising an inside 17b defining an opening 17c that accommodates the rim 20, wherein the first toothed portion 19a and the second toothed portion 19b of the spring member 17 are arranged on the inside 17b and protrude radially inwards to face the rim 20. As indicated in Figs. 5a and 5b the opening 17c comprises a first inner diameter D1 and a second inner diameter D2 perpendicular to the first inner diameter D1, wherein the first and the second toothed portions 19a, 19b of the spring member 17 face the toothed portion 20a of the rim 20 in the direction of the first inner diameter D 1, wherein when the control members 8a, 8b reside in the first positions, the first inner diameter D1 is smaller than the second inner diameter D2 and comprises a magnitude that ensures that the toothed portions 19a, 19b engage with the toothed portion 20a of the rim 20 (cf. Fig. 5a), and wherein when the control members 8a, 8b are moved into their second positions, the spring member 17 is deformed so that the first inner diameter D1 is increased to disengage the toothed portions 19a, 19b of the spring member 17 and the toothed portion 20a of the rim 20. Due to this deformation of the spring member 17, the second inner diameter D2 is decreased, but does not prevent rotation of the spring member 17 about rotation axis z.

Particularly, as shown e.g. in Figs. 5a and 5b, the control member(s) 8a, 8b is/are configured to be pushed radially inwards from the first position to the second position to deform the annular part 17a of the spring member 17 so that the first inner diameter D1 increases and the second inner diameter decreases D2. Particularly, the annular part 17a of the spring member 17 can e.g. be shaped elliptically, wherein the toothed portions 19a, 19b face each other in the direction of the small semi-axis A of the annular part 17a of the spring member 17 (cf. also Fig. 4) along which the first diameter D1 extends. Further, the opposing guide pins 18 can face each other in said direction of the small semi-axis A, too. However, the control members 8a, 8b protrude radially outwards from the annular part 17a of the spring member 17 in the direction of the large semi-axis A' corresponding to the second diameter D2. Thus, due to pressing onto the control member(s) 8a, 8b, the annular part 17a can be elastically deformed into a circular shape (or less elliptical shape) which causes disengagement of the toothed portions 19a, 19b of the spring member 17 from the toothed portion 20a of the rim 20 as shown in Fig. 5b. However, other shapes of the annular part 17a / spring member 17 that allow sufficient increase of the first diameter D1 are also conceivable.

When manually pressing on the control members 8a, 8b (e.g. using index finger and thumb) the two control members 8a and 8b are guided by the slots 15 of the knob 7. At the same time, the guide pins (e.g. cylinders or cuboids) 18 ensure via their guided movement in the grooves 16 of the rotating cap 7 that the spring member 17 remains centered.

Fig. 6 shows the cross-section A-A through the handle housing 4, 5 shown in Fig. 3. The actuating member 200 carries a second and a third deflection element 24b, 24a. The deflection elements 24b, 24a can be pulleys supported on pins 27b, 27a. Furthermore, the deflection elements 24b, 24a can be arranged between the upper plate 21 and the lower plate 23. Particularly, upper and lower plate 21, 23 can be connected via the pins 27b, 27a which can protrude from the lower plate 23 into holes formed in the upper plate 21.

When the actuating member 200 is not locked and rotated about rotation axis z, it takes the deflection elements 24b, 24a along which are both arranged spaced apart from the rotation axis z. Particularly, the actuating member 200 can be rotated about the rotation axis z by approx. +/-40°. Furthermore, the two deflection elements (e.g. pulleys) 24b and 24a can simultaneously act as spacers between upper 21 and lower plate 23. Particularly, enough space is provided between the lower plate 23 and the handle base 5 for accommodating electrode lines 26 and the optional rinsing hose 9. Particularly, the upper plate 21, is rotatably mounted on the handle cover 4 to rotatably support the actuating member 200 on the base 5 (via cover 4). The knob 7 can be clipped into the opening 22 of the upper plate 21 via the snap hooks 14. By operating the control members 8a and/or 8b, the described locking mechanism is unlocked and the knob 7 and with it the upper 21 and lower plate 23 can be rotated by +/- 40° with their pulleys 24a and 24b.

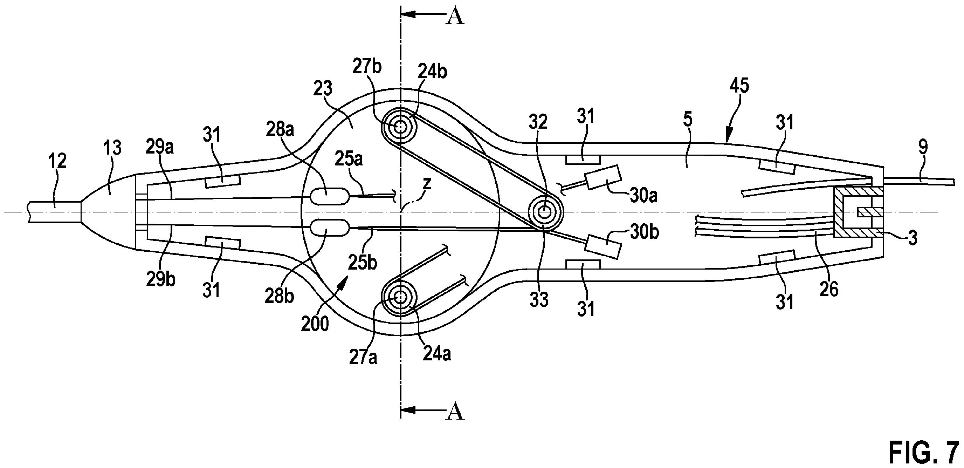

Fig. 7 shows a view onto the handle base 5 (e.g. before mounting the upper plate 21 and the handle cover 4). Also indicated are elongated first and second pulling members 29b, 29a, wherein the first pulling member 29b is used for generating the left deflection of the deflectable section 11 of the shaft 12 in the first deflection direction L and the second pulling wire 29a is used for generating the right deflection in the second deflection direction R (cf. also Fig. 2). Particularly, the pulling members for the left- 29b and right-deflection 29a enter the handle or handle base 5 from the shaft 12. Because of the symmetry of the mechanism for left and right deflection, the following description can be limited to one side, here for left deflection. For the sake of clarity, some elements for right deflection are therefore only shown in abbreviated form by break lines.

In the handle 45, the pulling members (e.g. pulling wires) 29b, 29a for the left deflection and right deflection can comprise more bending flexible sections with high extension stiffness (e.g. ropes with high young modulus) 25b and 25a, respectively, that can be connected to remaining (e.g. stiffer) sections of the pulling members 29b, 29a by means of crimps 28b and 28a as shown in Fig. 7. Particularly, the first pulling member 29b - particularly pulling rope 25b - for the exemplarily considered left deflection is guided to the second deflection member (e.g. pulley) 24b of the actuating member 200 via a first deflection element (e.g. pulley) 33 arranged on the handle base 5. From there, the first pulling member 29b (e.g. rope 25b) is guided back again, past the first deflection element 33, into an anchoring 30b for the left deflection pulling member 29b (e.g. rope 25b) on the handle base 5. The same procedure is followed for components 24a, 30a and 29a (25a). Particularly, the upper plate 21, not shown here, can be placed with its holes on the pins 27a and 27b of the lower plate 23. Then, the handle cover 4, which is also not shown here, is connected to the base 5 (e.g. snapped into the snap-in pockets 31 of the handle base 5) after a functional test of the catheter 1.

Fig. 8 shows the same view as in Fig. 7, but with complete left deflection (see dashed lines in Fig. 2). Furthermore, the configuration shown also occurs during the functional test just before the handle assembly is completed. By rotating the actuating member 200 comprising the lower plate 23 and the deflection elements 24b, 24a arranged thereon by about 40° to the stop, the second deflection element 24b is moved away from the first deflection element 33. Returning the first pulling member 29b (particularly pulling rope 25b) from the second deflection element 24b to the anchoring 30b, the first pulling member 29b (particularly pulling rope 25b) is pulled twice as far as the second deflection member 24b (pin 27b) has moved away from the first deflection member 33 / bearing pin 32. Thus, the transmission ratio is 1:2.

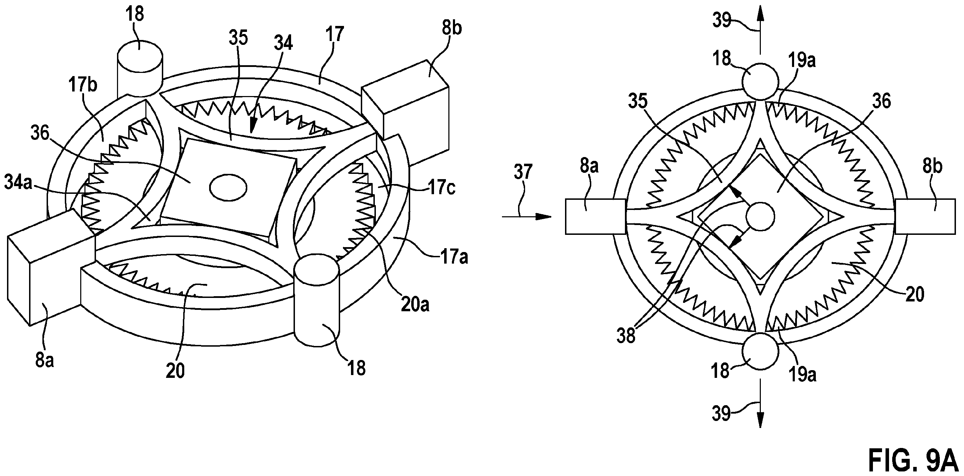

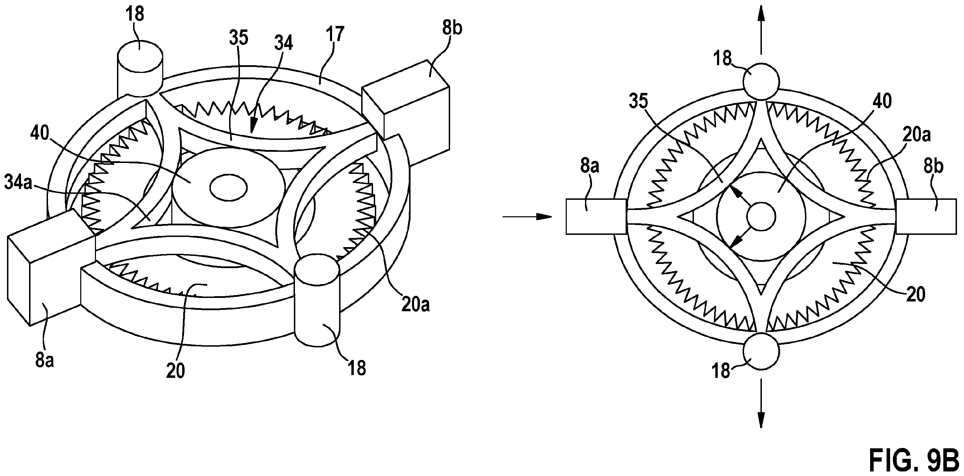

Figs. 9a and 9b show yet another embodiment of the present invention based on the spring member 17. Here, the spring member 17 comprises - besides the annular part 17a - a force deflection structure 34, that can comprise four arc-shaped force deflection struts 35. These struts 35 in interaction with a polygonal protrusion 36 or a circular or elliptical deflection protrusion 40 can be used to amplify the action of the control members 8a and 8b such that actuation 37 of only one of the members (e.g. 8a) alone is sufficient to lift the toothed portions 19a, 19b outwards 39 from the toothed portion 20a of the rim 20. When one of the control members (e.g. 8a) is actuated, the deflection protrusions 36 or 40 generate spreading counterforces 38 on the force deflection struts 35 and thus on the respective short half-axis of the annular spring part 17a. Particularly, the protrusion 36, 40 can be connected to the upper plate 21 or form an integral part thereof.

Fig. 10 shows yet another embodiment of the spring member 17 with a first and a second toothed portion 19a, 19b. Particularly, the toothed portions 19a, 19b each comprise a plurality of sawtooth-shaped teeth for the left deflection (first toothed portion 19a) of section 11 of shaft 12 in the first deflection direction L and the right deflection of section 11 in the second deflection direction R (second toothed portion 19b), cf. also Fig. 2.

Here, the toothed portion 20a of the rim 20 comprises two adjacent (e.g. quarter) segments 201, 202 particularly comprising sawtooth-shaped outer teeth for selectively engaging with the first toothed portion 19a for the left deflection and the second toothed portion 19b for the right deflection of the deflectable section 11 of the shaft of catheter 1. In the case of a left deflection (rotation of spring member 17 in the first rotation direction 41a), the first toothed portion 19a engages solely with the first segment 201 after the control members 8a and 8b are released, whereas the second toothed portion 19b is free and without contact with the rim 20. The reverse is correspondingly true for the right deflection (rotation of spring member 17 in the second rotation direction 41b). Here, the second toothed portion 19b solely engages with the second segment 202 after release of the control members 8a, 8b. With this embodiment, tooth pairings with dimensions in the submillimeter range can be realized, as is common with cable ties.

An alternative embodiment is shown in Fig. 10 where wedge clamps 46, 45 are used instead of toothed portions 19a, 19b of the spring element 17 and the corresponding toothed portions 20a, 20b of the rim 20. This alternative embodiment is shown in the same view as the previous embodiment is shown in Fig. 4. The wedges 45 engage with the wedge slots 46 in the locked state. Thereby the surface of the wedges 45 and/or the wedge slots 46 comprise a rough or structured surface to provide a friction lock or form lock between the wedge 45 and the wedge slot 46 in the locked state, e.g. until the spring member 17' is deformed by a slight pressure applied to the first and/or second control member 8a, 8b. In this embodiment only a slight deformation of the spring member 17' by the control members 8a and/or 8b is necessary to unlock the friction or (micro)form lock between the wedges 45 and the wedge lock 46.

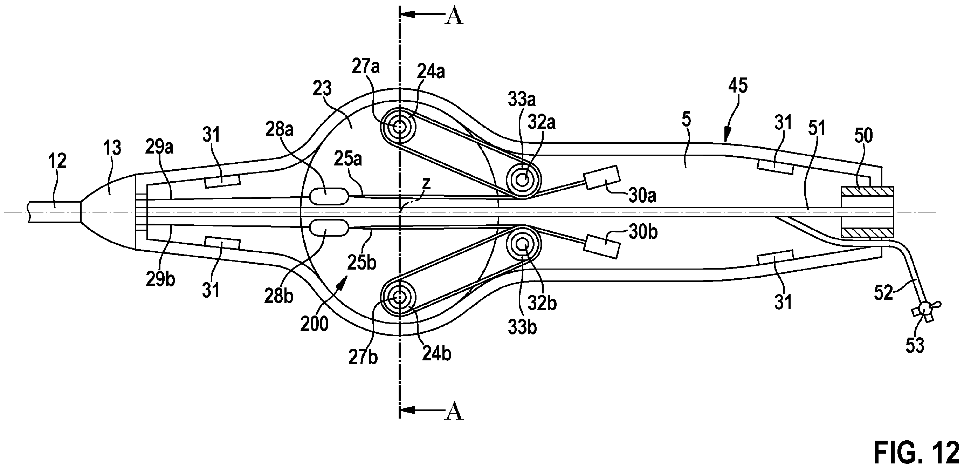

Another alternative embodiment is shown in Fig. 12. Fig. 12 shows a fcross-sectional view along the handle of an alternative arrangement of the course of elongated pulling members that couple the actuating member to the deflectable section suitable for guiding shafts. According to this embodiment the first pulling member 29b (e.g. first pulling rope 25b) and the second pulling member 29a (e.g. second pulling rope 25a) are connected to two separate first deflection elements 30a and 30b. Thereby a guide tube 51 can be arranged along the longitudinal axis of the handle. The guide tube 51 together haemostatic valve 50 and the separate irrigation lumen 52 connected to a 3-way valve 53 would allow the use of the handle in connection with a steerable introducer.

Particularly, the present invention offers the advantage that a lateral actuating pressure on one or both control members 8a, 8b is already sufficient to unlock the locking mechanism. Particularly, the locking mechanism can consist of three simple injection molded parts, namely the handle cover 4 comprising the rim 20, the spring member 17 comprising the control member(s) 8a, 8b and particularly a rotating knob 7. Furthermore, advantageously, the locking mechanism is located outside the closed and thus splash-proof handle housing. It can be clipped into the cable pull gear after the handle cover. Further, the cable pull gear can comprise a 1:2 ratio and the anchorings of the pulling members are proximal to the control members, so that the handle housing can be shortened distally and the proximal space in the handle housing can be placed under the palm of the user's hand. Furthermore, the configuration of the pulling member gear according to the invention has the advantage that the length of the released pulling member is greater than the pull path of the pulling member. This avoids compression of the catheter and overloading of the transmission due to double pulling effects.

Further, a continuous cylindrical pin in the axis of rotation of the pulling member gear is not required. This gives the pulling members and their crimps sufficient freedom of movement. Furthermore, all media, such as electrode lines and, if necessary, flushing hoses, are routed in the handle base under the pulling member gear. Here, too, no cylindrical pin interferes.

Furthermore, the complete mechanical and electrical functionality, as well as the adjustment function of the catheter are connected to the handle base and can be checked and adjusted even before the handle cover is clipped on. Finally, particularly, neither adhesive nor screw connections are required. If the handle has to be opened again for reworking, only the snap-in hooks of the rotating knob or the handle cover may be damaged, which would then have to be replaced.

## Claims

1. A catheter (1), comprising:
- a shaft (12) having a deflectable section (11), and
- a handle (45) connected to the shaft (12), wherein the handle (45) comprises a rim (20) having a toothed portion (20a) and a rotatable actuating member (200) configured to be rotated to deflect the deflectable section (11), wherein the handle (45) further comprises a first control member (8a) coupled to a spring member (17) having a first toothed portion (19a) configured to engage with the toothed portion (20a) of the rim (20) to prevent rotation of the actuating member (200), and wherein the first control member (8a) is configured to be manually moved from a first position to a second position to deform the spring member (17) such that the first toothed portion (19a) of the spring member (17) disengages with the toothed portion (20a) of the rim (20) to allow rotation of the actuating member (200) for deflecting the deflectable section (11) of the shaft (12).

2. The catheter according to claim 1, wherein the spring member (17) comprises an annular part (17a) comprising an inside (17b) defining an opening (17c) for receiving the rim (20), and wherein the first toothed portion (19a) of the spring member (17) is arranged on the inside (17b) and protrudes inwards.

3. The catheter according to one of the preceding claims, wherein the opening (17c) comprises a first inner diameter (D1) and a second inner diameter (D2) perpendicular to the first inner diameter (D1), wherein the first toothed portion (19a) of the spring member (17) faces the toothed portion (20c) of the rim (20) in the direction of the first inner diameter (D1), wherein when the first control member (8a) resides in the first position, the first inner diameter (D1) is smaller than the second inner diameter (D2), and wherein when the first control member (8a) resides in the second position, the spring member (17) is deformed so that the first inner diameter (D1) is increased to disengage the first toothed portion (19a) of the spring member (17) and the toothed portion (20a) of the rim (20) and the second inner diameter (D2) is decreased.

4. The catheter according one of the preceding claims, wherein the first control member (8a) is coupled to the spring member (17) and is configured to be pushed radially inwards from the first position to the second position to deform the annular member so that the first inner diameter (D1) increases and the second inner diameter decreases (D2).

5. The catheter according to claim 2 or according to one of the claims 3 to 4 insofar referring to claim 2, wherein when the first control member (8a) resides in the first position, the inside (17b) comprises a non-circular oval shape, particularly an elliptical shape, and wherein when the first control member (8a) resides in the second position, the inside (17b) is deformed to comprise a circular shape.

6. The catheter according to one of the preceding claims, wherein the handle (45) comprises a second control member (8b) coupled to the spring member (17), wherein the spring member (17) comprises a second toothed portion (19b) configured to engage with the toothed portion (20a) of the rim (20) to prevent rotation of the actuating member (200), and wherein the second control member (8b) is configured to be manually moved from a first position to a second position at the same time as the first control member is moved from its first position to its second position to deform the spring member (17) such that the first and the second toothed portion (19a, 19b) of the spring member (17) both disengage with the toothed portion (20a) of the rim (20) for allowing rotation of the actuating member (200) for deflecting the deflectable section (11) of the shaft (12).

7. The catheter according to one of the claims 1 to 5, wherein the handle (45) comprises a second control member (8b) coupled to the spring member (17), wherein the spring member (17) comprises a second toothed portion (19b), and wherein the toothed portion (20a) of the rim (20) merely extends over a section of a periphery of the rim (20) and is comprised of a first segment (201) associated to the first toothed portion (19a) of the spring member (17) and an adjacent second segment (202) associated to the second toothed portion (19b) of the spring member (17), wherein the first toothed portion (19a) is configured to engage with the first segment (201) to prevent rotation of the actuating member (200) in a first rotation direction (41a), wherein the second toothed portion (19b) is configured to engage with the second segment (202) to prevent rotation of the actuating member (200) in an opposite second rotation direction (41b), and wherein the second control member (8b) is configured to be manually moved from a first position to a second position at the same time as the first control member (8a) is moved from its first position to its second position to deform the spring member (17) such that the first toothed portion (19a) disengages with the first segment (201) or such that the second toothed portion (19b) disengages with the second segment (202) of the toothed portion (20a) of the rim (20) for allowing rotation of the actuating member (200) for deflecting the deflectable section (11) of the shaft (12).

8. The catheter according to claim 6 or 7, wherein the second control member (8b) is arranged opposite the first control member (8a) and/or wherein the second toothed portion (19b) of the spring member (17) is arranged opposite the first toothed portion (19a).

9. The catheter according to claim 2 and one of the claims 6 to 8, wherein the spring member (17) further comprises a force deflection structure (34) connected to the inside (17b) of the annular part (17a), wherein the force deflection structure (34) is configured to transmit a force introduced into the spring member (17) via one of the control members (8a, 8b) to the first and/or second toothed portion (19a, 19b) of the spring member (17).

10. The catheter according to one of the preceding claims, wherein the force deflection structure (34) contacts a protrusion (36, 40) received in a through-opening (34a) of the force deflection structure (34), wherein the protrusion (36, 40) is connected to the actuating member (200) in a rotationally fixed manner.

11. The catheter according to one of the preceding claims, wherein the spring member (17) comprises two opposing guide pins (18) for guiding the deformation of the spring member (17).

12. The catheter according to one of the preceding claims, wherein the handle (45) comprises an actuating knob (7) coupled to the actuating member (200) for manually rotating the actuating member (200), wherein particularly the knob (7) comprises a first slot (15) receiving the first control member (8a) and an opposing second slot receiving the second control member (8b), and wherein particularly the knob (7) comprises two opposing grooves (16), each groove (16) receiving one of the guide pins (18) so that the respective guide pin (18) can slide in the respective groove (16) when the first and/or second control member (8a, 8b) is moved from its first position to its second position and vice versa.

13. The catheter according to one of the preceding claims, wherein the catheter (1) further comprises an elongated first pulling member (29b) connected to the deflectable section (11), wherein the actuating member (200) is rotatable about a rotation axis (z) with respect to a base (5) of the handle (45), and wherein the handle (45) further comprises a first deflection element (33) arranged on the base (5) and a second deflecting element (24b) arranged on the rotatable actuating member (200) spaced apart from the rotation axis (z), wherein the first pulling member (29b) coming from the deflectable section (11) is deflected by the first deflection element (33) towards the second deflection element (24b) that deflects the first pulling member (29b) towards a first anchoring (30b) of the first pulling member (29b) on the base (5), so that a rotation of the actuating member (200) in a first rotation direction (41a) about the rotation axis (z) increases a distance between the first and the second deflection element (33, 24b) and generates a pulling force on the first pulling member (29b) thereby causing a deflection of the deflectable section (11) of the shaft (12) in a first deflection direction (L).

14. A catheter (1), comprising:
- a shaft (12) having a deflectable section (11),
- an elongated first pulling member (29b) connected to the deflectable section (11),
- a handle (45) connected to the shaft (12), wherein the handle (45) comprises a rotatable actuating member (200) being rotatable about a rotation axis (z) with respect to a base (5) of the handle (45), and wherein the handle (45) further comprises a first deflection element (33) arranged on the base (5) and a second deflection element (24b) arranged on the rotatable actuating member (200) spaced apart from the rotation axis (z), wherein the first pulling member (29b) coming from the deflectable section (11) is deflected by the first deflection element (33) towards the second deflection element (24b) that deflects the first pulling element (29b) towards an anchoring (30b) of the first pulling member (29b) on the base (5), so that a rotation of the actuating member (200) in a first direction (41a) about the rotation axis (z) increases a distance between the first and the second deflection element (33, 24b) and generates a pulling force on the first pulling member (29b) thereby causing a deflection of the deflectable section (11) in a first deflection direction (L).

15. The catheter according to claim 14, wherein the handle (45) further comprises a rim (20) having a toothed portion (20a) and a first control member (8a) coupled to a spring member (17) having a first toothed portion (19a) configured to engage with the toothed portion (20a) of the rim (20) to prevent rotation of the actuating member (200), and wherein the first control member (8a) is configured to be manually moved from a first position to a second position to deform the spring member (17) such that the first toothed portion (19a) of the spring member (17) disengages with the toothed portion (20a) of the rim (20) to allow rotation of the actuating member (200) for deflecting the deflectable section (11) of the shaft (12).
